# EUROPEAN PATENT SPECIFICATION

(11) **EP 3 841 952 B1**
(45) Date of publication and mention of the grant of the patent: **20.11.2024**
(21) Application number: 19851041.4
(22) Date of filing: 17.07.2019
(51) Int. Cl.: A61B 1/045, G02B 23/24, A61B 1/005

(54) **IMAGE PROCESSING APPARATUS, IMAGE PROCESSING METHOD, AND IMAGE PROCESSING PROGRAM**
BILDVERARBEITUNGSVORRICHTUNG, BILDVERARBEITUNGSVERFAHREN UND BILDVERARBEITUNGSPROGRAMM
APPAREIL DE TRAITEMENT D'IMAGE, PROCÉDÉ DE TRAITEMENT D'IMAGE ET PROGRAMME DE TRAITEMENT D'IMAGE

(30) Priority: 24.08.2018 JP 2018157428
(43) Date of publication of application: 30.06.2021
(73) Proprietor: FUJIFILM Corporation, Tokyo 106-8620 (JP)
(72) Inventor: KAGAYA, Makoto, Ashigarakami-gun, Kanagawa 258-8538 (JP)
(74) Representative: Dehns Germany Partnerschaft mbB
(86) International application number: PCT/JP2019/028074
(87) International publication number: WO 2020/039800

(56) References cited:
- EP-A1- 1 504 712
- EP-A1- 2 827 793
- EP-B1- 2 827 793
- WO-A1-2017/170842
- WO-A1-2017/170842
- WO-A1-2018/116573
- WO-A1-2018/135041
- JP-A- 2001 046 318
- JP-A- 2004 358 095
- JP-A- 2017 169 910
- JP-A- H08 107 875

## Description

### BACKGROUND OF THE INVENTION

### 1. Field of the Invention

The present disclosure relates to an image processing device, an image processing method, and an image processing program.

### 2. Description of the Related Art

In the related art, in an examination of the inside of the body of a subject by an endoscope (hereinafter, referred to as an "endoscopic examination"), the endoscopic examination may be supported by detecting the shape of an insertion part of an endoscope inserted into the body of the subject and displaying a shape image showing the shape of the insertion part on a display unit.

For example, JP2006-296576A discloses a technique for combining an endoscopic image obtained by an endoscope and a shape image and displaying the combined image on a display unit. In addition, for example, JP2000-079088A discloses a technique for displaying the shape of an insertion part of an endoscope in a case of being observed in a predetermined viewpoint direction, and the shape of the insertion part in a viewpoint direction different by 90 degrees from the viewpoint direction on a display unit. An image processing device according to the preamble of claim 1 is known from EP2827793 B1.

### SUMMARY OF THE INVENTION

However, in the techniques disclosed in JP2006-296576A and JP2000-079088A, although the shape image is displayed, it may be insufficient as a display for observing a predetermined shape as the shape of the insertion part, for example, a so-called loop shape in which an examiner wants to observe the state. Therefore, it may be difficult for the examiner to grasp the shape of the insertion part of the endoscope.

The present disclosure has been made in view of the above circumstances, and an object of the present disclosure is to provide an image processing device, an image processing method, and an image processing program capable of easily grasping the shape of an insertion part of an endoscope.

In order to achieve the above object, there is provided an image processing device according to a first aspect of the present disclosure. The image processing device comprises: an acquisition unit that acquires a shape of an insertion part to be inserted into a subject in an endoscope; a storage unit in which a predetermined shape is stored; a determination unit that determines whether or not the shape of the insertion part includes the predetermined shape stored in the storage unit; and a display controller that controls a display unit to display a first shape image in a case where determination is made that the shape of the insertion part does not include the predetermined shape, and controls the display unit to display the first shape image and a second shape image in a case where determination is made that the shape of the insertion part includes the predetermined shape, the first shape image representing the shape of the insertion part in a first viewpoint direction, the second shape image including a portion corresponding to the predetermined shape of the insertion part in a second viewpoint direction different from the first viewpoint direction.

An image processing device according to a second aspect of the present disclosure, in the image processing device according to the first aspect, further comprises a generation unit that generates the first shape image in a case where the determination unit determines that the shape of the insertion part does not include the predetermined shape, and generates the first shape image and the second shape image in a case where the determination unit determines that the shape of the insertion part includes the predetermined shape, the first shape image representing the shape of the insertion part in the first viewpoint direction, the second shape image including a portion corresponding to the predetermined shape of the insertion part in the second viewpoint direction different from the first viewpoint direction.

In an image processing device according to a third aspect of the present disclosure, in the image processing device according to the first or second aspect, the predetermined shape is a loop shape.

In an image processing device according to a fourth aspect of the present disclosure, in the image processing device according to the third aspect, the second viewpoint direction is a direction of connection from an intersection point of a loop-shaped portion of the insertion part to a center point of the loop-shaped portion.

In an image processing device according to a fifth aspect of the present disclosure, in the image processing device according to the third aspect, the second viewpoint direction is a predetermined direction as a viewpoint direction for grasping an intersecting state of a loop-shaped portion of the insertion part.

In an image processing device according to a sixth aspect of the present disclosure, in the image processing device according to any one of the first to fifth aspects, in a case where the determination unit determines that the shape of the insertion part includes the predetermined shape, the display controller controls the display unit to display the second shape image on a partial region in the first shape image.

In an image processing device according to a seventh aspect of the present disclosure, in the image processing device according to any one of the first to sixth aspects, in a case where the determination unit determines that the shape of the insertion part includes the predetermined shape, the display controller controls the display unit to further display information that is visually recognizable in the second viewpoint direction.

In an image processing device according to an eighth aspect of the present disclosure, in the image processing device according to any one of the first to seventh aspects, in a case where the determination unit determines that the shape of the insertion part includes the predetermined shape, the display controller performs control such that the second shape image in which the second viewpoint direction is changed is displayed for each changed second viewpoint direction.

In an image processing device according to a ninth aspect of the present disclosure, in the image processing device according to any one of the first to seventh aspects, in a case where the shape of the insertion part includes the predetermined shape, the display controller performs control such that the second shape image in which the portion corresponding to the predetermined shape of the insertion part is rotated is displayed for each rotated rotation position.

In an image processing device according to a tenth aspect of the present disclosure, in the image processing device according to any one of the first to ninth aspects, the first viewpoint direction is a predetermined direction as a viewpoint direction for grasping an overall shape of the insertion part.

In an image processing device according to an eleventh aspect of the present disclosure, in the image processing device according to any one of the first to tenth aspects, the display controller controls the display unit to further display an endoscopic image obtained by the endoscope.

An image processing device according to a twelfth aspect of the present disclosure, in the image processing device according to any one of the first to eleventh aspects, further comprises a detection unit that detects the shape of the insertion part to be inserted into the subject in the endoscope, and the acquisition unit acquires the shape of the insertion part from the detection unit.

There is provided an image processing method according to a thirteenth aspect of the present disclosure. The image processing method executed by a computer, comprises: acquiring a shape of an insertion part to be inserted into a subject in an endoscope; acquiring a predetermined shape stored in a storage unit; determining whether or not the shape of the insertion part includes the predetermined shape; and controlling a display unit to display a first shape image in a case where determination is made that the shape of the insertion part does not include the predetermined shape, and controlling the display unit to display the first shape image and a second shape image in a case where determination is made that the shape of the insertion part includes the predetermined shape, the first shape image representing the shape of the insertion part in a first viewpoint direction, the second shape image including a portion corresponding to the predetermined shape of the insertion part in a second viewpoint direction different from the first viewpoint direction.

There is provided an image processing program according to a fourteenth aspect of the present disclosure. The image processing program causes a computer to execute a process comprising: acquiring a shape of an insertion part to be inserted into a subject in an endoscope; acquiring a predetermined shape stored in a storage unit; determining whether or not the shape of the insertion part includes the predetermined shape; and controlling a display unit to display a first shape image in a case where the shape of the insertion part does not include the predetermined shape, and controlling the display unit to display the first shape image and a second shape image in a case where the shape of the insertion part includes the predetermined shape, the first shape image representing the shape of the insertion part in a first viewpoint direction, the second shape image including a portion corresponding to the predetermined shape of the insertion part in a second viewpoint direction different from the first viewpoint direction.

In addition, the image processing device according to the aspects of the present disclosure is an image processing device including a processor. The processor acquires a shape of an insertion part to be inserted into a subject in an endoscope, controls a display unit to display a first shape image in a case where the shape of the insertion part does not include a predetermined shape, and controls the display unit to display the first shape image and a second shape image in a case where the shape of the insertion part includes the predetermined shape, the first shape image representing the shape of the insertion part in a first viewpoint direction, the second shape image including a portion corresponding to the predetermined shape of the insertion part in a second viewpoint direction different from the first viewpoint direction.

According to the present disclosure, it is possible to easily grasp the shape of the insertion part of the endoscope.

### BRIEF DESCRIPTION OF THE DRAWINGS

Fig. 1 is a configuration diagram showing an example of a configuration of an endoscope system of an embodiment.
Fig. 2 is a block diagram showing an example of a configuration of the endoscope system of the embodiment.
Fig. 3 is a configuration diagram showing an example of a receiving coil unit and a transmitting coil unit of a position detection device of the embodiment.
Fig. 4 is a block diagram showing an example of a configuration of an image processing unit of the embodiment.
Fig. 5 is a block diagram showing an example of a hardware configuration of the image processing unit, controllers, and a hardware configuration of the embodiment.
Fig. 6 is a flowchart showing an example of image processing performed by the image processing unit of the embodiment.
Fig. 7 is an explanatory diagram illustrating a first shape image generated by a generation unit of the image processing unit of the embodiment.
Fig. 8 is a diagram showing an example of a combined image including an endoscopic image and the first shape image displayed on a display unit.
Fig. 9 is an explanatory diagram illustrating a second viewpoint direction of a second shape image generated by the generation unit of the image processing unit of the embodiment.
Fig. 10 is an explanatory diagram illustrating the second shape image generated by the generation unit of the image processing unit of the embodiment.
Fig. 11 is a diagram showing an example of a combined image including the endoscopic image, the first shape image, and the second shape image displayed on the display unit.
Fig. 12 is a diagram showing another example of the combined image including the endoscopic image, the first shape image, and the second shape image displayed on the display unit.
Fig. 13 is a diagram showing another example of the combined image including the endoscopic image, the first shape image, and the second shape image displayed on the display unit.

### DESCRIPTION OF THE PREFERRED EMBODIMENTS

Hereinafter, examples of an embodiment for implementing a technique of the present disclosure will be described in detail with reference to the drawings.

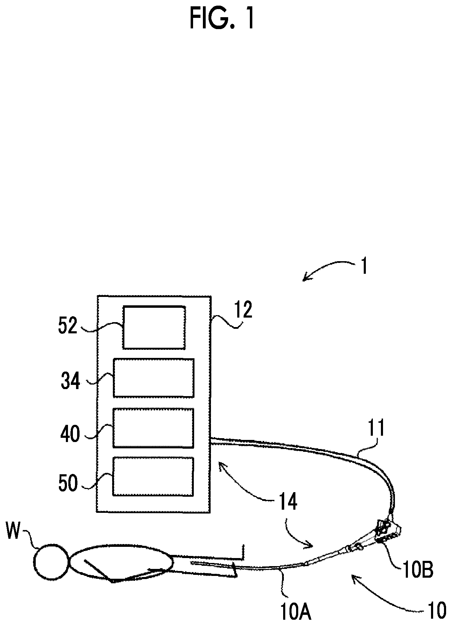

First, the overall configuration of an endoscope system 1 of the present embodiment will be described with reference to Fig. 1. Fig. 1 is a configuration diagram showing an example of a configuration of the endoscope system 1 of the present embodiment.

The endoscope system 1 comprises an endoscope 10 that captures an image of the inside of the body of a subject W (hereinafter, referred to as an "endoscopic image"), an endoscopic examination device 12, and a position detection device 14.

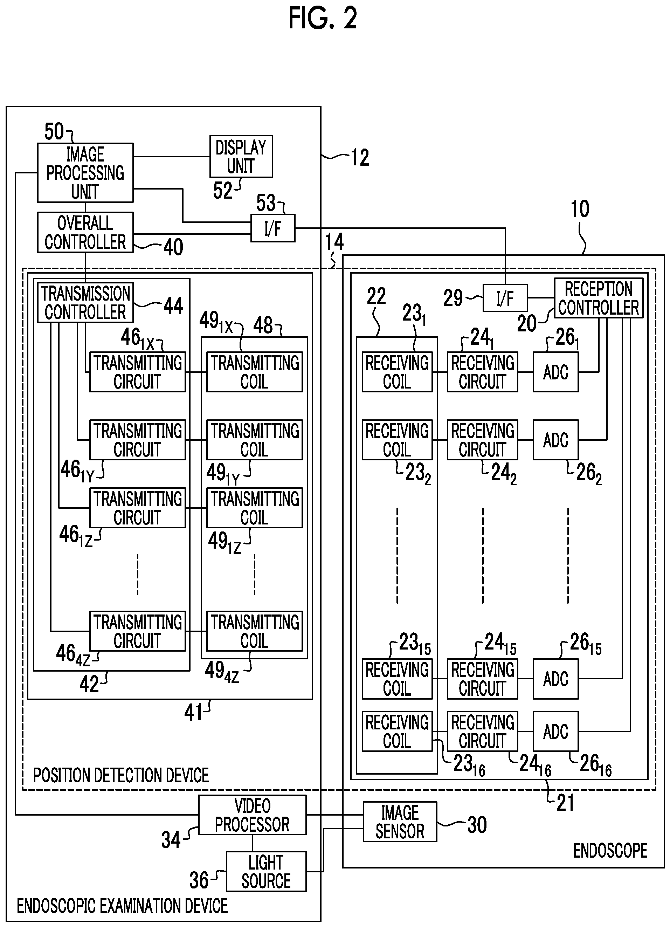

The endoscope 10 comprises an insertion part 10A and an operation part 10B, and in a case where an endoscopic examination is performed, an examiner operates the operation part 10B to insert the insertion part 10A into the body of the subject W, and captures an endoscopic image of the inside of the body of the subject W. The endoscopic examination device 12 connected to the endoscope 10 by a cable 11 comprises a video processor 34, an overall controller 40, an image processing unit 50, and a display unit 52 such as a liquid crystal display. The video processor 34 controls capturing of an endoscopic image by the endoscope 10. The overall controller 40 controls the entire endoscope system 1. The image processing unit 50 performs image processing on various images displayed on the display unit 52. The image processing unit 50 of the present embodiment is an example of an image processing device of the present disclosure. On the other hand, the position detection device 14 comprises a transmission unit 41 (see Fig. 2) provided in the endoscopic examination device 12 and a reception unit 21 (see Fig. 2) provided inside the endoscope 10, and detects a position of the insertion part 10A by receiving a magnetic field generated by the transmission unit 41 at the reception unit 21.

Next, with reference to Fig. 2, detailed configurations of the endoscope 10, the endoscopic examination device 12, and the position detection device 14 will be described. Fig. 2 is a block diagram showing an example of the configuration of the endoscope system 1 of the present embodiment.

As shown in Fig. 2, the endoscope 10 comprises an image sensor 30 including an imaging element such as a charge coupled device (CCD) image sensor and a complementary metal-oxide-semiconductor (CMOS) image sensor. Under the control of the video processor 34, the endoscope 10 transmits light emitted from a light source 36 through a transmission path (not shown), emits the light from an emitting portion (not shown) provided at the distal end of the insertion part 10A, and illuminates the inside of the body of the subject W with the emitted light. Reflected light from the subject W due to this illumination light forms an image on the image sensor 30 by an objective lens (not shown), an image signal corresponding to an endoscopic image, which is a formed optical image, is output to the video processor 34 of the endoscopic examination device 12 via the cable 11. The video processor 34 performs predetermined image processing on the input image signal, and image data of the endoscopic image obtained by this image processing is output to the image processing unit 50.

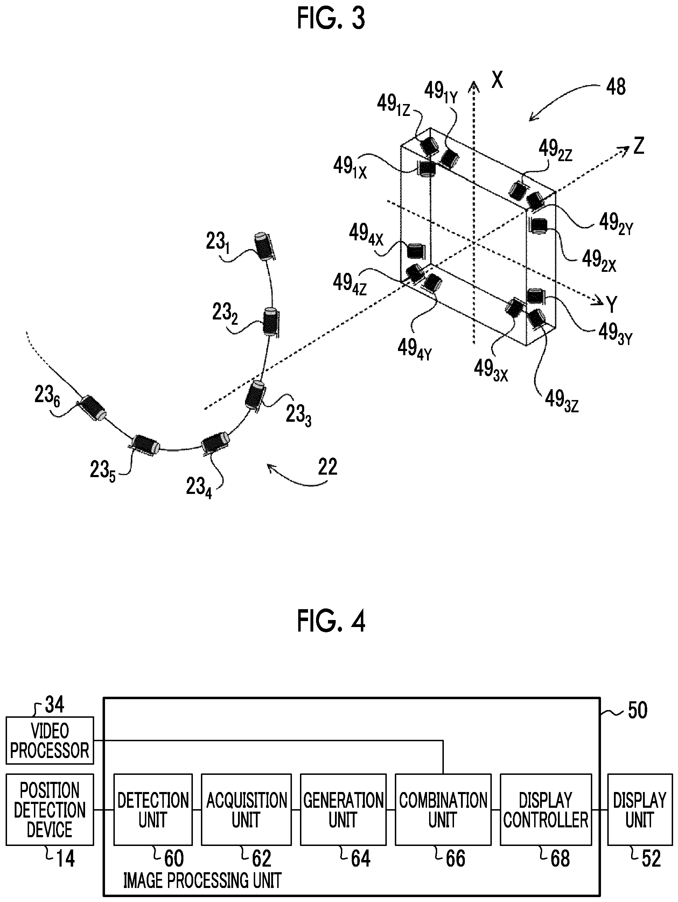

As shown in Fig. 2, in the position detection device 14, the transmission unit 41 provided inside the endoscopic examination device 12 comprises a transmission control unit 42 and a transmitting coil unit 48. As shown in Fig. 3, the transmitting coil unit 48 comprises a plurality (12 in the present embodiment) of transmitting coils 49, specifically, transmitting coils 49_{1X}, 49_{1Y}, 49_{1Z}, 49_{2X}, 49_{2Y}, 49_{2Z}, 49_{3X}, 49_{3Y}, 49_{3Z}, 49_{4X}, 49_{4Y}, and 49_{4Z}. In the present embodiment, in the case where the transmitting coils 49 are collectively referred to, the transmitting coils 49 are simply referred to as the "transmitting coil 49", and in the case of distinguishing each, reference numerals (_{1X}, ..., _{4Z}) representing each are added after the "transmitting coil 49".

As shown in Fig. 3, the transmitting coil 49 of the present embodiment includes, as a set, three transmitting coils 49 whose axes are oriented in the directions of an X-axis, a Y-axis, and a Z-axis, respectively, and the transmitting coil unit 48 comprises four sets of transmitting coil groups. Specifically, the transmitting coil unit 48 comprises a set of the transmitting coil 49_{1X} oriented in the X-axis direction, the transmitting coil 49_{1Y} oriented in the Y-axis direction, and the transmitting coil 49_{1Z} oriented in the Z-axis direction, and a set of the transmitting coil 49_{2X} oriented in the X-axis direction, the transmitting coil 49_{2Y} oriented in the Y-axis direction, and the transmitting coil 49_{2Z} oriented in the Z-axis direction. In addition, the transmitting coil unit 48 comprises a set of the transmitting coil 49_{3X} oriented in the X-axis direction, the transmitting coil 49_{3Y} oriented in the Y-axis direction, and the transmitting coil 49_{3Z} oriented in the Z-axis direction, and a set of the transmitting coil 49_{4X} oriented in the X-axis direction, the transmitting coil 49_{4Y} oriented in the Y-axis direction, and the transmitting coil 49_{4Z} oriented in the Z-axis direction. As described above, the transmitting coil unit 48 of the present embodiment is equivalent to a state where four triaxial coils are provided as the transmitting coils 49.

In addition, the transmission control unit 42 comprises a transmission controller 44 and a transmitting circuit 46 connected to the transmitting coil 49, specifically, transmitting circuits 46_{1X}, 46_{1Y}, 46_{3Z}, 46_{2X}, 46_{2Y}, 46_{2Z}, 46_{3X}, 46_{3Y}, 46_{3Z}, 46_{4X}, 46_{4Y}, and 46_{4Z.} In the present embodiment, similarly to the transmitting coil 49, in the case where the transmitting circuits 46 are collectively referred to, the transmitting circuits 46 are simply referred to as the "transmitting circuit 46", and in the case of distinguishing each, reference numerals (_{1X}, ..., _{4Z}) representing each are added after the "transmitting circuit 46".

The transmitting circuit 46 generates a drive signal for driving the transmitting coil 49 under the control of the transmission controller 44, and outputs the drive signal to the transmitting coil 49 connected thereto. A drive signal is applied to each transmitting coil 49 such that the transmitting coil 49 radiates an electromagnetic wave accompanied by a magnetic field to the surroundings. The transmission controller 44 of the present embodiment causes each transmitting circuit 46 to generate a drive signal and drive each transmitting coil 49 sequentially at a predetermined time interval, for example, at an interval of several tens of milliseconds.

On the other hand, as shown in Fig. 2, in the position detection device 14, the reception unit 21 provided inside the endoscope 10 comprises a reception controller 20, a receiving coil unit 22, receiving circuits 24 (24₁ to 24₁₆), analog-to-digital converters (ADCs) 26 (26₁ to 26₁₆), and an interface (I/F) 29. The reception controller 20 controls the entire reception unit 21 and controls the driving of the receiving coil unit 22.

As shown in Fig. 3, the receiving coil unit 22 comprises, for example, 16 (six shown in Fig. 3) receiving coils 23, specifically, receiving coils 23₁ to 23₁₆. In the present embodiment, similarly to the transmitting coil 49, in the case where the receiving coils 23, the receiving circuits 24, and the ADCs 26 are collectively referred to, the receiving coils 23, the receiving circuits 24, and the ADCs 26 are simply referred to as the "receiving coil 23", the "receiving circuit 24", and the "ADC 26", respectively, and in the case of distinguishing each, reference numerals (₁, ..., ₁₆) representing each are added after the "receiving coil 23", the "receiving circuit 24", and the "ADC 26".

Each of the receiving coils 23 of the receiving coil unit 22 is arranged in the insertion part 10A of the endoscope 10 along the direction of insertion into the subject W. The receiving coil 23 detects a magnetic field generated by each transmitting coil 49 of the transmitting coil unit 48. Each receiving coil 23 is connected to the receiving circuit 24, and outputs a detection signal corresponding to the detected magnetic field to the receiving circuit 24. The receiving circuit 24 includes a low pass filter (LPF), an amplifier (both not shown), and the like, the disturbance noise is removed by the LPF, and the detection signal amplified by the amplifier is output to the ADC 26. The ADC 26 converts the input analog detection signal into a digital detection signal and outputs the digital detection signal to the reception controller 20. The reception controller 20 transmits the detection signal input from each ADC 26 to the endoscopic examination device 12 via the I/F 29.

The detection signal input to the endoscopic examination device 12 is input to the image processing unit 50 via an I/F 53.

The image processing unit 50 detects the position of each receiving coil 23 based on the input detection signal. That is, the image processing unit 50 of the present embodiment detects the magnetic field generated by each transmitting coil 49 with the receiving coil 23, and detects the position and direction (orientation) of each receiving coil 23 based on the detection signal output from the receiving coil 23. The method by which the image processing unit 50 detects the position of the receiving coil 23 based on the detection signal is not particularly limited, and, for example, a technique disclosed in JP3432825B can be applied thereto. In the technique disclosed in JP3432825B, from a measured value of the magnetic field generated by each transmitting coil 49 and an estimated value in the direction of the receiving coil 23, an estimated value of the distance from the specific transmitting coil 49 to the receiving coil 23 is calculated. Next, an estimated value of the position of the receiving coil 23 is calculated from the estimated value of the distance from each transmitting coil 49 to the receiving coil 23 and the known position of the transmitting coil 49. Subsequently, a new estimated value in the direction of the receiving coil 23 is calculated from the estimated position of the receiving coil 23 and a measured value of the magnetic field of the receiving coil 23. Then, the position and direction of the receiving coil 23 are derived by repeating the calculation of the estimated value of the distance from the transmitting coil 49 to the receiving coil 23 and the calculation of the estimated value of the position of the receiving coil 23 described above using the new estimated value in the direction of the receiving coil 23.

Further, the image processing unit 50 of the present embodiment detects the shape of the insertion part 10A of the endoscope 10 based on the detected position and direction of each receiving coil 23. Fig. 4 is a functional block diagram showing an example of the image processing unit 50 of the present embodiment.

As shown in Fig. 4, the image processing unit 50 of the present embodiment comprises a detection unit 60, an acquisition unit 62, a generation unit 64, a combination unit 66, and a display controller 68. The detection signal is input to the detection unit 60 from the position detection device 14, specifically, from the reception controller 20 of the endoscope 10 via the I/F 29 and the I/F 53. The detection unit 60 detects the position and direction of each receiving coil 23 based on the input detection signal. Further, the detection unit 60 detects the shape of the insertion part 10A based on the detected position and direction of the receiving coil 23, and outputs information indicating the detected shape and the position and direction of each receiving coil 23 to the acquisition unit 62. The acquisition unit 62 acquires information indicating the shape of the insertion part 10A and the position and direction of each receiving coil 23 from the detection unit 60, and outputs the information to the generation unit 64.

The generation unit 64 generates a first shape image (details described later) representing the shape of the insertion part 10A in a first viewpoint direction (details described later) based on the position and direction of each receiving coil 23, and outputs image data of the first shape image to the combination unit 66. In addition, based on the information indicating the shape of the insertion part 10A, the generation unit 64 further generates a second shape image (details described later) including a predetermined shape (details described later) of the insertion part 10A in a second viewpoint direction (details described later) different from the first viewpoint direction in a case where the shape of the insertion part 10A includes the predetermined shape, and outputs image data of the second shape image to the combination unit 66.

Image data of an endoscopic image is input from the video processor 34 to the combination unit 66. In addition, the image data of the first shape image, or the image data of the first shape image and the image data of the second shape image are input from the generation unit 64. The combination unit 66 generates image data of a combined image obtained by combining at least one of the image data of the first shape image or the image data of the second shape image, which is input, with the image data of the endoscopic image, and outputs the generated image data to the display controller 68.

The display controller 68 controls the display unit 52 to display an image represented by the image data output from the combination unit 66. That is, in a case where the shape of the insertion part 10A does not include the predetermined shape, the display controller 68 of the present embodiment causes the display unit 52 to display the first shape image representing the shape of the insertion part 10A in the first viewpoint direction. Further, in a case where the shape of the insertion part 10A includes the predetermined shape, the display controller 68 controls the display unit 52 to display the first shape image and the second shape image.

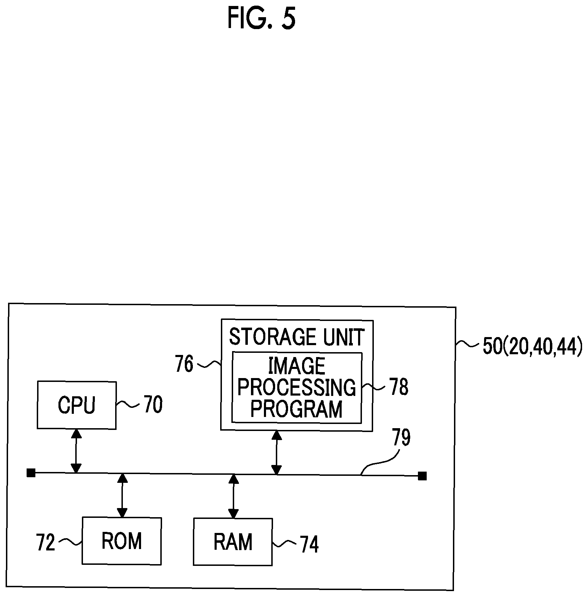

As an example, the image processing unit 50 of the present embodiment is realized by a microcomputer or the like including the hardware shown in Fig. 5. As shown in Fig. 5, the image processing unit 50 comprises a central processing unit (CPU) 70, a read only memory (ROM) 72, a random access memory (RAM) 74, and a nonvolatile storage unit 76 such as a hard disk drive (HDD), a solid state drive (SSD), and a flash memory. The CPU 70, the ROM 72, the RAM 74, and the storage unit 76 are connected to a bus 79 such that they can communicate with each other. The storage unit 76 stores an image processing program 78 for performing image processing whose details will be described later. The CPU 70 reads out the image processing program 78 from the storage unit 76, loads the read-out program in the RAM 74, and executes the loaded image processing program 78. The CPU 70 executes the image processing program 78, and thus, the image processing unit 50 functions as each of the detection unit 60, the acquisition unit 62, the generation unit 64, the combination unit 66, and the display controller 68.

In addition, in the endoscope system 1 of the present embodiment, the reception controller 20, the overall controller 40, and the transmission controller 44 are also realized by the same hardware as the image processing unit 50 (see Fig. 5).

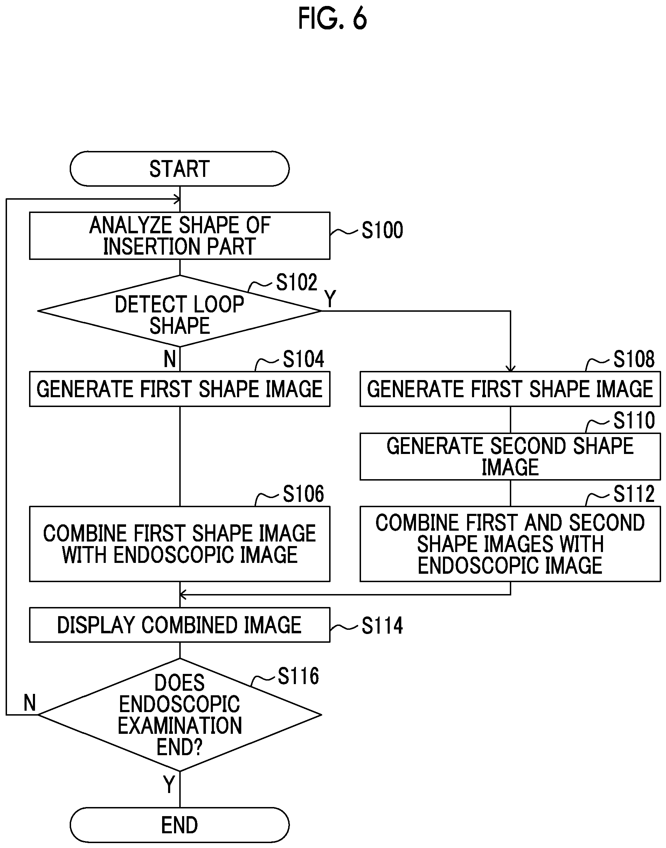

Next, the operation of the image processing unit 50 of the present embodiment will be described. Fig. 6 is a flowchart showing an example of a flow of image processing performed by the CPU 70 of the image processing unit 50. As an example, in the endoscope system 1 of the present embodiment, in a case where an instruction to start the endoscopic examination is received from an examiner by operating an operation button or the like (not shown), the CPU 70 executes the image processing program 78, and thus the image processing shown in Fig. 6 is performed.

In step S100, the detection unit 60 analyzes the shape of the insertion part 10A. In the present embodiment, the detection unit 60 first detects the position and direction of each receiving coil 23 based on the detection signal input from the position detection device 14 as described above. Then, the shape of the insertion part 10A is analyzed based on the detected position and direction of the receiving coil 23, and in the shape of the insertion part 10A, a loop shape is detected as an example of a predetermined shape. In the present embodiment, so-called α loop shape and inverse α loop shape are detected as an example of the loop shape. The method by which the detection unit 60 detects the loop shape is not particularly limited, and, for example, a technique disclosed in JP2004-358095A or a technique disclosed in JP2008-119259A can be applied thereto.

The acquisition unit 62 acquires information indicating the shape of the insertion part 10A and the position and direction of each receiving coil 23 from the detection unit 60, and outputs the information to the generation unit 64.

In the next step S102, the generation unit 64 determines whether or not the detection unit 60 has detected a loop shape, in other words, whether or not the shape of the insertion part 10A includes a loop shape. In a case where the loop shape is not detected, the determination in step S102 is a negative determination, and the process proceeds to step S104. The generation unit 64 of the present embodiment is an example of a determination unit of the present disclosure. The generation unit 64 can acquire the predetermined shape from the storage unit 76 that stores the predetermined shape such as the loop shape, and determine whether or not the shape detected by the detection unit 60 includes the predetermined shape. Further, the detection unit 60 may be an example of the determination unit. In this case, the detection unit 60 may acquire the predetermined shape from the storage unit 76 and detect the predetermined shape from the shape of the insertion part 10A to determine whether or not the shape of the insertion part 10A includes the predetermined shape.

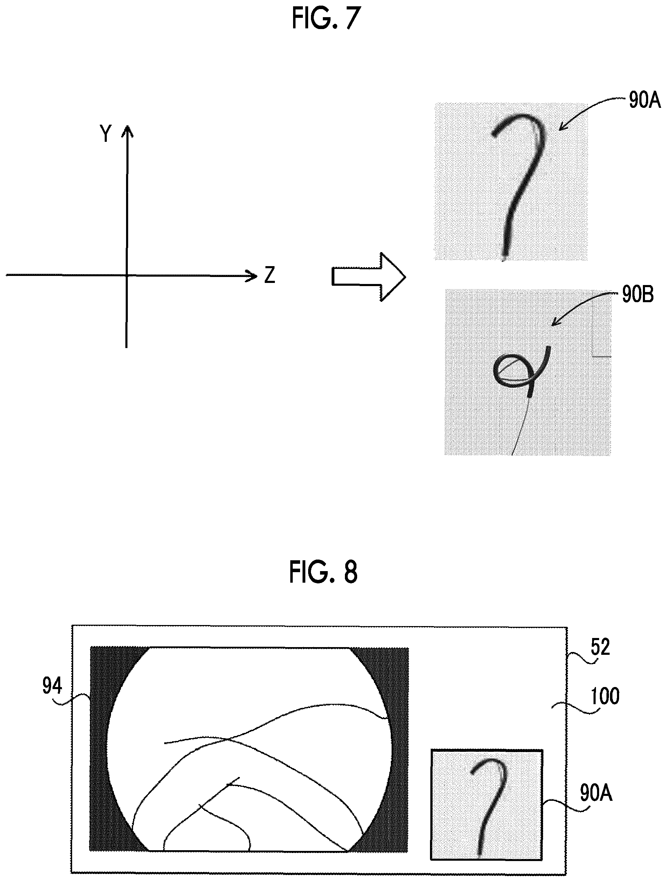

In step S104, as described above, the generation unit 64 generates the first shape image representing the shape of the insertion part 10A in the first viewpoint direction based on the position and direction of each receiving coil 23, and outputs image data of the first shape image to the combination unit 66. As an example, the first viewpoint direction in the present embodiment is a predetermined direction as a viewpoint direction for grasping the overall shape of the insertion part 10A. A specific example of such a first viewpoint direction includes the X-axis direction in the transmitting coil unit 48 shown in Fig. 3, and in the present embodiment, a direction in which the examiner looks at the subject W in front (a direction in which the face-side surface is visually recognized) can be included. In this case, first shape images 90A and 90B representing the shape of the insertion part 10A in the first viewpoint direction are two-dimensional images represented by the Y-axis and the Z-axis, as shown in Fig. 7. The first shape image 90A shown in Fig. 7 is the first shape image in the case where the loop shape is not included, and the first shape image 90B shown in Fig. 7 is the first shape image in the case where the loop shape is included. In the following, in a case where the first shape image 90A and the first shape image 90B are collectively referred to, the first shape image 90A and the first shape image 90B are simply referred to as the "first shape image 90", and in the case of distinguishing each, reference numerals (A, B) representing each are added after the "first shape image 90". In addition, another specific example of the first viewpoint direction includes a direction in which it is possible to visually recognize that the insertion part is annular (forms a closed curve) in the case where the insertion part has a loop shape.

In the next step S106, the combination unit 66 generates an image in which the first shape image 90A and the endoscopic image are combined, and outputs image data of the combined image including the first shape image 90A and the endoscopic image to the display controller 68. In the next step S114, the display controller 68 causes the display unit 52 to display the combined image. Fig. 8 shows an example of a combined image 100 displayed on the display unit 52. The combined image 100 shown in Fig. 8 includes the first shape image 90A and the endoscopic image 94. Note that, in the present embodiment, an image combined in a state where the first shape image 90A and the endoscopic image 94 are arranged side by side is used as the combined image 100. However, the method of combining the first shape image 90A and the endoscopic image 94 is not limited to the present embodiment. The degree of superimposition of the first shape image 90A and the endoscopic image 94 in the combined image 100, the size of each image, and the like may be in a state where the image desired by the examiner is appropriately displayed in the endoscopic examination. For example, the combined image 100 may be an image combined in a state where the first shape image 90A and the endoscopic image 94 are at least partially superimposed. Further, the combined image 100 may be in a form in which the degree of superimposition of the first shape image 90A and the endoscopic image 94 and the size of each image are controlled according to the size of the display unit 52 and the like.

On the other hand, in a case where the loop shape is detected, the determination in step S102 is a positive determination, and the process proceeds to step S108. In step S108, the generation unit 64 generates the first shape image 90B in the same manner as the generation of the first shape image 90A in step S104.

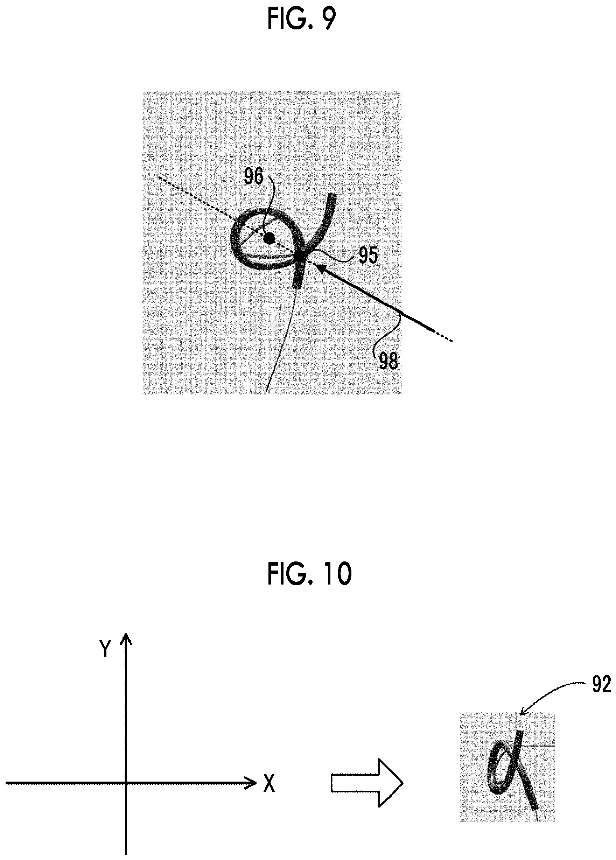

In the next step S110, the generation unit 64 generates the second shape image representing the shape of the insertion part 10A in the second viewpoint direction based on the position and direction of each receiving coil 23. As an example, the second viewpoint direction in the present embodiment is a predetermined direction as a viewpoint direction for grasping the intersecting state of the loop-shaped portion. As a specific example of such a direction, in the present embodiment, as shown in Fig. 9, a direction of connection from a loop-shaped intersection point 95 to a loop-shaped center point 96 is defined as a second viewpoint direction 98. In this case, a second shape image 92 representing the shape of the insertion part 10A in the second viewpoint direction 98 is a two-dimensional image represented by the Y-axis and the X-axis, as shown in Fig. 10. By setting the second viewpoint direction to the second viewpoint direction 98 in this way, it is possible to easily grasp the shape (state) of the loop shape in the depth direction.

As an example, in the present embodiment, the second shape image 92 is not an image representing the overall shape of the insertion part 10A, but is an image mainly representing only the loop-shaped portion as shown in Fig. 10. Note that, an image is not limited to the second shape image 92 shown in Fig. 10, and may be an image representing the overall shape of the insertion part 10A similarly to the first shape image 90. However, by using an image mainly representing only the loop-shaped portion as in the present embodiment, it is easy to grasp the state of the loop shape, particularly the intersecting portion of the loop shape. In addition, the second shape image 92 of the present embodiment is set as an image in which the size of the loop shape is larger than the size of the loop shape in the first shape image 90. For example, the size of the loop-shaped image in the second shape image 92 is set to be four times the size of the loop-shaped image in the first shape image 90 (each side of the rectangle is twice). In this way, by setting the second shape image 92 as an image mainly representing only the loop shape, it is possible to use the image in a state where the loop shape can be easily grasped while suppressing an increase in the overall size of the second shape image 92.

The image data of the first shape image 90B and the image data of the second shape image 92 which are generated in this way are output to the combination unit 66.

In the next step S 112, the combination unit 66 generates an image in which the first shape image 90B, the second shape image 92, and the endoscopic image are combined, and outputs image data of the combined image 100 including the first shape image 90B, the second shape image 92, and the endoscopic image to the display controller 68. In the next step S 114, the display controller 68 causes the display unit 52 to display the combined image.

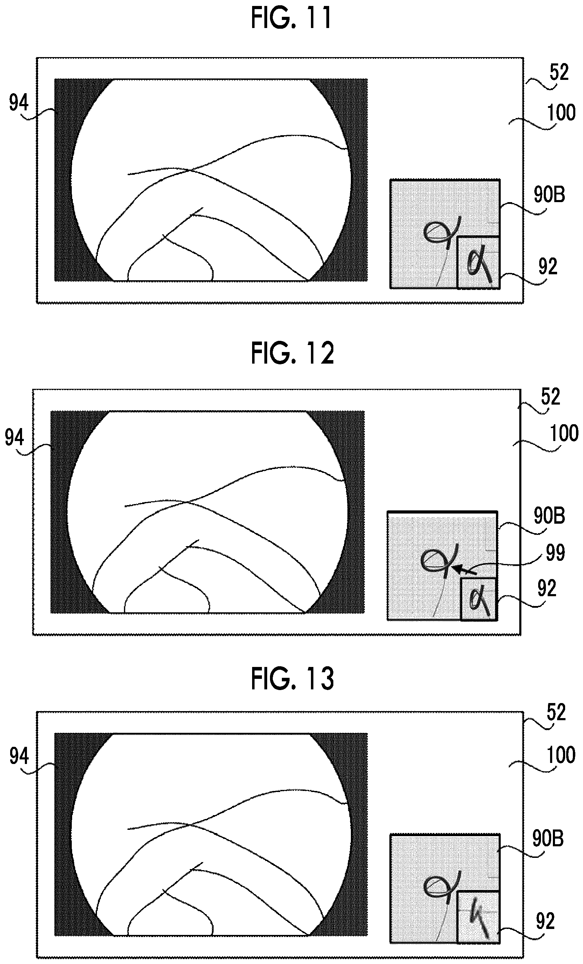

Fig. 11 shows an example of the combined image 100 displayed on the display unit 52. The combined image 100 shown in Fig. 11 includes the first shape image 90B, the second shape image 92, and the endoscopic image 94. Note that, in the present embodiment, an image which is combined in a state where the second shape image 92 is superimposed on a partial region in the first shape image 90B, and then, further combined in a state where the combined image and the endoscopic image 94 are arranged side by side is used as the combined image 100. However, the method of combining the first shape image 90B, the second shape image 92, and the endoscopic image 94 is not limited to the present embodiment. The degree of superimposition of the first shape image 90B, the second shape image 92, and the endoscopic image 94 in the combined image 100, the size of each image, and the like may be in a state where the image desired by the examiner is appropriately displayed in the endoscopic examination. For example, the combined image 100 may be an image combined in a state where the first shape image 90B and the second shape image 92 are arranged side by side. Further, for example, the combined image 100 may be an image combined in a state where the first shape image 90B, the second shape image 92, and the endoscopic image 94 are at least partially superimposed. Further, the combined image 100 may be in a form in which the degree of superimposition of the first shape image 90B, the second shape image 92, and the endoscopic image 94 and the size of each image are controlled according to the size of the display unit 52 and the like.

Note that, as shown in Fig. 12, it is preferable that the generation unit 64 also generate viewpoint information 99 as information that can be visually recognized in the second viewpoint direction 98, and the display controller 68 cause the display unit 52 to display the viewpoint information 99. Regarding the display of the viewpoint information 99, the display position, the shape, the size, and the like are not particularly limited. However, as in the example shown in Fig. 12, the viewpoint direction can be more easily grasped by superimposing the viewpoint information 99 having the shape of an arrow indicating the viewpoint direction on the first shape image 90B and displaying it.

Further, the generation unit 64 may generate, as the second shape image 92, the second shape image 92 in which the second viewpoint direction is changed for each changed second viewpoint direction, and the display controller 68 may cause the display unit 52 to display the generated second shape image 92. In addition, for example, the second shape image 92 may be the second shape image 92 in a state where the loop shape is rotated. In this case, the generation unit 64 may set the rotation speed per rotation to 4 seconds to 10 seconds every predetermined time, for example, clockwise or counterclockwise and generate a predetermined number of second shape images 92 such as 2 images to 6 images per rotation for each rotation position, and the display controller 68 may cause the display unit 52 to display the second shape image 92. Fig. 13 shows an example of the combined image 100 displayed on the display unit 52 in this case. In this case, the second shape image 92 displayed on the display unit 52 changes with time, for example, from the second shape image 92 shown in Fig. 11 to the second shape image 92 shown in Fig. 13. Note that, the storage unit 76 may store rotation display information such as the orientation of rotation (clockwise or counterclockwise), the rotation speed (time required for one rotation), and the number of images per rotation of the second shape image for each predetermined shape, the generation unit 64 may generate the second shape image 92 based on the rotation display information acquired from the storage unit 76, and the display controller 68 may cause the display unit 52 to display the generated second shape image 92.

In the next step S 116, the display controller 68 determines whether to end the endoscopic examination. In the endoscope system 1 of the present embodiment, by operating an operation button or the like (not shown), the determination in step S 116 is a negative determination until an instruction to end the endoscopic examination is received from an examiner by operating an operation button or the like (not shown), the process returns to step S 100, and the respective processes of steps S 102 to S 114 are repeated. On the other hand, in a case where the instruction to end the endoscopic examination is received, the determination in step S 116 is a positive determination, and the present image processing ends.

As described above, the image processing unit 50 of the present embodiment comprises the acquisition unit 62 and the display controller 68. The acquisition unit 62 acquires the shape of the insertion part 10A to be inserted into the subject W in the endoscope 10. In a case where the shape of the insertion part 10A does not include a loop shape that is a predetermined shape, the display controller 68 controls the display unit 52 to display the first shape image 90A representing the shape of the insertion part 10A in the first viewpoint direction. Further, in a case where the shape of the insertion part 10A includes the predetermined shape, the display controller 68 controls the display unit 52 to display the first shape image 90B and the second shape image 92 including a portion corresponding to the predetermined shape of the insertion part 10A in the second viewpoint direction different from the first viewpoint direction.

In this way, the image processing unit 50 of the present embodiment displays the first shape image 90B and the second shape image 92 in a case where the loop shape is included. Since the second shape image 92 is the shape of the insertion part 10A in the second viewpoint direction, which is different from the first viewpoint direction, it is possible to easily grasp the loop shape, particularly in the present embodiment, the overlapping (intersection) state of the loop. Further, according to the image processing unit 50 of the present embodiment, in a case where the loop shape is not included, since only the first shape image 90A is displayed, the shape of the insertion part 10A can be more easily grasped by the first shape image 90A.

Therefore, according to the image processing unit 50 of the present embodiment, it is possible to easily grasp the shape of the insertion part 10A of the endoscope 10.

Further, according to the image processing unit 50 of the present embodiment, even in a case where the endoscopic image 94, the first shape image 90B, and the second shape image 92 are displayed on the display unit 52, the loop shape of the insertion part 10A can be easily grasped by the second shape image 92. Moreover, according to the image processing unit 50 of the present embodiment, since the loop-shaped image in the second shape image 92 is larger than the loop-shaped image included in the first shape image 90B, the loop shape can be more easily grasped.

In the present embodiment, the loop shape, particularly the α loop shape and the inverse α loop shape are used as an example of the predetermined shape; however, the predetermined shape is not limited to these shapes, and the shape may be any shape that the examiner desires to check in detail the state of the shape of the insertion part 10A. For example, it may be another loop shape such as an N loop shape or a γ loop shape, or may be a shape according to individual settings of the examiner.

In addition, although the position detection device 14 of the present embodiment has the configuration in which the transmission unit 41 including the transmitting coil unit 48 that generates a magnetic field is arranged in the endoscopic examination device 12, and the reception unit 21 including the receiving coil unit 22 that detects the magnetic field is arranged in the endoscope 10, the position detection device 14 is not limited to the configuration of the present embodiment. For example, an element that generates a magnetic field other than the transmitting coil unit 48 (transmitting coil 49) such as a spin torque oscillator may be used. Further, for example, an element that detects a magnetic field other than the receiving coil unit 22 (receiving coil 23) such as a Hall element or a magneto resistive (MR) element may be used.

The position detection device 14 may have a configuration in which the reception unit 21 is arranged in the endoscopic examination device 12 and the transmission unit 41 is arranged in the endoscope 10. Further, the position detection device 14 may be a device that detects the shape of the insertion part 10A using a magnetic field other than the magnetic field. For example, the position detection device 14 may be a device that uses an optical cable or the like in which a fiber bragg grating (FBG) is arranged and that detects the shape of the insertion part 10A by the FBG

Further, in the present embodiment, the configuration in which the image processing unit 50 has the functions of the detection unit 60, the acquisition unit 62, the generation unit 64, the combination unit 66, and the display controller 68 has been described; however, some of these functions may be provided by another device or a plurality of devices. For example, one of the detection unit 60 and the generation unit 64 may be provided in a device external to the image processing unit 50.

Further, in the present embodiment, the form in which the combination unit 66 generates the combined image 100 obtained by combining the first shape image 90 and the endoscopic image 94 has been described; however, the present disclosure is not limited to the present embodiment, and the first shape image 90 and the endoscopic image 94 may be displayed on the display unit 52 as separate images without being combined. In addition, each of the endoscopic image 94, the first shape image 90, and the second shape image 92 is not limited to the same display unit 52, and may be displayed on different display devices, for example. Alternatively, the display device that displays the endoscopic image 94 and the display device that displays the first shape image 90 and the second shape image 92 may have different forms.

In the present embodiment, as hardware structures of processing units that execute various kinds of processing, such as the detection unit 60, the acquisition unit 62, the generation unit 64, the combination unit 66, and the display controller 68, various processors shown below can be used.

As described above, the various processors include a programmable logic device (PLD) as a processor of which the circuit configuration can be changed after manufacture, such as a field programmable gate array (FPGA), a dedicated electrical circuit as a processor having a dedicated circuit configuration for executing specific processing such as an application specific integrated circuit (ASIC), and the like, in addition to the CPU as a general-purpose processor that functions as various processing units by executing software (program).

One processing unit may be configured by one of the various processors, or configured by a combination of the same or different kinds of two or more processors (for example, a combination of a plurality of FPGAs or a combination of the CPU and the FPGA). In addition, a plurality of processing units may be configured by one processor.

As an example where a plurality of processing units are configured by one processor, first, there is a form in which one processor is configured by a combination of one or more CPUs and software as typified by a computer, such as a client or a server, and this processor functions as a plurality of processing units. Second, there is a form in which a processor for realizing the function of the entire system including a plurality of processing units by one integrated circuit (IC) chip as typified by a system on chip (SoC) or the like is used. As described above, various processing units are configured by using one or more of the above-described various processors as hardware structures.

Furthermore, as the hardware structure of the various processors, more specifically, an electrical circuit (circuitry) in which circuit elements such as semiconductor elements are combined can be used.

In the above embodiment, the image processing program 78 is described as being stored (installed) in the storage unit 76 in advance; however, the present disclosure is not limited thereto. The image processing program 78 may be provided in a form recorded in a recording medium such as a compact disk read only memory (CD-ROM), a digital versatile disk read only memory (DVD-ROM), and a universal serial bus (USB) memory. Further, the image processing program 78 may be downloaded from an external device via a network.

### Explanation of References

1: endoscope system
10: endoscope
10A: insertion part
10B: operation part
11: cable
12: endoscopic examination device
14: position detection device
20: reception controller
21: reception unit
22: receiving coil unit
23₁ to 23₁₆: receiving coil
24₁ to 24₁₆: receiving circuit
26₁ to 26₁₆: ADC
29: I/F
30: image sensor
34: video processor
36: light source
40: overall controller
41: transmission unit
42: transmission control unit
44: transmission controller
46_{1X}, 46_{1Y}, 46_{1Z} to 46_{4X}, 46_{4Y}, 46_{4Z}: transmitting circuit
48: transmitting coil unit
49_{1X}, 49_{1Y}, 49_{1Z} to 49_{4X}, 49_{4Y}, 49_{4Z}: transmitting coil
50: image processing unit
52: display unit
53: I/F
60: detection unit
62: acquisition unit
64: generation unit
66: combination unit
68: display controller
70: CPU
72: ROM
74: RAM
76: storage unit
78: image processing program
79: bus
90, 90A, 90B: first shape image
92: second shape image
94: endoscopic image
95: intersection point
96: center point
98: second viewpoint direction
99: viewpoint information
100: combined image

## Claims

1. An image processing device comprising:
an acquisition unit (62) that acquires a shape of an insertion part (10A) to be inserted into a subject in an endoscope (10);
a storage unit (76) in which a predetermined shape is stored;
a determination unit that determines whether or not the shape of the insertion part (10A) includes the predetermined shape stored in the storage unit (76); and **characterised by**
a display controller (68) that controls a display unit (52) to display a first shape image in a case where the determination unit determines that the shape of the insertion part (10A) does not include the predetermined shape, and controls the display unit (52) to display the first shape image and a second shape image in a case where the determination unit determines that the shape of the insertion part (10A) includes the predetermined shape, the first shape image representing the shape of the insertion part (10A) in a first viewpoint direction, the second shape image including a portion corresponding to the predetermined shape of the insertion part (10A) in a second viewpoint direction different from the first viewpoint direction.

2. The image processing device according to claim 1, further comprising:
a generation unit (64) that generates the first shape image in a case where the determination unit determines that the shape of the insertion part (10A) does not include the predetermined shape, and generates the first shape image and the second shape image in a case where the determination unit determines that the shape of the insertion part (10A) includes the predetermined shape, the first shape image representing the shape of the insertion part (10A) in the first viewpoint direction, the second shape image including a portion corresponding to the predetermined shape of the insertion part (10A) in the second viewpoint direction different from the first viewpoint direction.

3. The image processing device according to claim 1 or 2, wherein the predetermined shape is a loop shape.

4. The image processing device according to claim 3, wherein the second viewpoint direction is a direction of connection from an intersection point of a loop-shaped portion of the insertion part (10A) to a center point of the loop-shaped portion.

5. The image processing device according to claim 3, wherein the second viewpoint direction is a predetermined direction as a viewpoint direction for grasping an intersecting state of a loop-shaped portion of the insertion part (10A).

6. The image processing device according to any one of claims 1 to 5, wherein in a case where the determination unit determines that the shape of the insertion part (10A) includes the predetermined shape, the display controller (68) controls the display unit (52) to display the second shape image on a partial region in the first shape image.

7. The image processing device according to any one of claims 1 to 6, wherein in a case where the determination unit determines that the shape of the insertion part (10A) includes the predetermined shape, the display controller (68) controls the display unit (52) to further display information that is visually recognizable in the second viewpoint direction.

8. The image processing device according to any one of claims 1 to 7, wherein in a case where the determination unit determines that the shape of the insertion part (10A) includes the predetermined shape, the display controller (68) performs control such that the second shape image in which the second viewpoint direction is changed is displayed for each changed second viewpoint direction.

9. The image processing device according to any one of claims 1 to 7, wherein in a case where the shape of the insertion part (10A) includes the predetermined shape, the display controller (68) performs control such that the second shape image in which the portion corresponding to the predetermined shape of the insertion part (10A) is rotated is displayed for each rotated rotation position.

10. The image processing device according to any one of claims 1 to 9, wherein the first viewpoint direction is a predetermined direction as a viewpoint direction for grasping an overall shape of the insertion part (10A).

11. The image processing device according to any one of claims 1 to 10, wherein the display controller (68) controls the display unit (52) to further display an endoscopic image obtained by the endoscope (10).

12. The image processing device according to any one of claims 1 to 11, further comprising:
a detection unit (60) that detects the shape of the insertion part (10A) to be inserted into the subject in the endoscope (10),
wherein the acquisition unit (62) acquires the shape of the insertion part (10A) from the detection unit (60).

13. An image processing method executed by a computer, comprising:
acquiring a shape of an insertion part (10A) to be inserted into a subject in an endoscope (10) from an acquisition unit (62);
acquiring a predetermined shape stored in a storage unit (76);
determining whether or not the shape of the insertion part (10A) includes the predetermined shape; and
controlling a display unit (52) to display a first shape image in a case where determination is made that the shape of the insertion part (10A) does not include the predetermined shape, and controlling the display unit (52) to display the first shape image and a second shape image in a case where determination is made that the shape of the insertion part (10A) includes the predetermined shape, the first shape image representing the shape of the insertion part (10A) in a first viewpoint direction, the second shape image including a portion corresponding to the predetermined shape of the insertion part (10A) in a second viewpoint direction different from the first viewpoint direction.

14. An image processing program for causing a computer to execute a process comprising:
acquiring a shape of an insertion part (10A) to be inserted into a subject in an endoscope (10) from an acquisition unit (62);
acquiring a predetermined shape stored in a storage unit (76);
determining whether or not the shape of the insertion part (10A) includes the predetermined shape; and
controlling a display unit (52) to display a first shape image in a case where determination is made that the shape of the insertion part (10A) does not include the predetermined shape, and controlling the display unit (52) to display the first shape image and a second shape image in a case where determination is made that the shape of the insertion part (10A) includes the predetermined shape, the first shape image representing the shape of the insertion part (10A) in a first viewpoint direction, the second shape image including a portion corresponding to the predetermined shape of the insertion part (10A) in a second viewpoint direction different from the first viewpoint direction.

## Patentansprüche

1. Vorrichtung zur Bildverarbeitung, umfassend:
eine Erfassungseinheit (62), die eine Form eines Einsteckteils (10A) erfasst, das in ein Subjekt in einem Endoskop (10) einzuführen ist;
eine Speichereinheit (76), in der eine vorbestimmte Form gespeichert ist;
eine Bestimmungseinheit, die bestimmt, ob die Form des Einsteckteils (10A) die in der Speichereinheit (76) gespeicherte vorbestimmte Form enthält oder nicht; und **gekennzeichnet durch**
eine Anzeigesteuerung (68), die eine Anzeigeeinheit (52) so steuert, dass sie ein erstes Formbild anzeigt, wenn die Bestimmungseinheit bestimmt, dass die Form des Einsteckteils (10A) die vorbestimmte Form nicht enthält, und die Anzeigeeinheit (52) so steuert, dass sie das erste Formbild und ein zweites Formbild anzeigt, wenn die Bestimmungseinheit bestimmt, dass die Form des Einsteckteils (10A) die vorbestimmte Form umfasst, wobei das erste Formbild die Form des Einsteckteils (10A) in einer ersten Beobachtungsrichtung repräsentiert, wobei das zweite Formbild einen Abschnitt umfasst, der der vorbestimmten Form des Einsteckteils (10A) in einer zweiten Beobachtungsrichtung entspricht, die sich von der ersten Beobachtungsrichtung unterscheidet.

2. Vorrichtung zur Bildverarbeitung nach Anspruch 1, die ferner umfasst:
eine Erzeugungseinheit (64), die das erste Formbild in einem Fall erzeugt, in dem die Bestimmungseinheit bestimmt, dass die Form des Einsteckteils (10A) die vorbestimmte Form nicht enthält, und das erste Formbild und das zweite Formbild in einem Fall erzeugt, in dem die Bestimmungseinheit bestimmt, dass die Form des Einsteckteils (10A) die vorbestimmte Form umfasst, wobei das erste Formbild die Form des Einsteckteils (10A) in der ersten Beobachtungsrichtung repräsentiert, wobei das zweite Formbild einen Abschnitt umfasst, der der vorbestimmten Form des Einsteckteils (10A) in der zweiten Beobachtungsrichtung entspricht, die sich von der ersten Beobachtungsrichtung unterscheidet.

3. Vorrichtung zur Bildverarbeitung nach Anspruch 1 oder 2, wobei die vorbestimmte Form eine Schleifenform ist.

4. Vorrichtung zur Bildverarbeitung nach Anspruch 3, wobei die zweite Beobachtungsrichtung eine Verbindungsrichtung von einem Schnittpunkt eines schleifenförmigen Abschnitts des Einsteckteils (10A) zu einem Mittelpunkt des schleifenförmigen Abschnitts ist.

5. Vorrichtung zur Bildverarbeitung nach Anspruch 3, wobei die zweite Beobachtungsrichtung eine vorbestimmte Richtung als Beobachtungsrichtung zum Erfassen eines Überschneidungszustands eines schleifenförmigen Abschnitts des Einsteckteils (10A) ist.

6. Vorrichtung zur Bildverarbeitung nach einem der Ansprüche 1 bis 5, wobei in einem Fall, in dem die Bestimmungseinheit bestimmt, dass die Form des Einsteckteils (10A) die vorbestimmte Form umfasst, die Anzeigesteuerung (68) die Anzeigeeinheit (52) so steuert, dass das zweite Formbild auf einem Teilbereich in dem ersten Formbild angezeigt wird.

7. Vorrichtung zur Bildverarbeitung nach einem der Ansprüche 1 bis 6, wobei in einem Fall, in dem die Bestimmungseinheit bestimmt, dass die Form des Einsteckteils (10A) die vorbestimmte Form enthält, die Anzeigesteuerung (68) die Anzeigeeinheit (52) steuert, um ferner Informationen anzuzeigen, die in der zweiten Beobachtungsrichtung visuell erkennbar sind.

8. Vorrichtung zur Bildverarbeitung nach einem der Ansprüche 1 bis 7, wobei in einem Fall, in dem die Bestimmungseinheit bestimmt, dass die Form des Einsteckteils (10A) die vorbestimmte Form enthält, die Anzeigesteuerung (68) eine Steuerung derart durchführt, dass das zweite Formbild, in dem die zweite Beobachtungsrichtung geändert ist, für jede geänderte zweite Beobachtungsrichtung angezeigt wird.

9. Vorrichtung zur Bildverarbeitung nach einem der Ansprüche 1 bis 7, wobei in einem Fall, in dem die Form des Einsteckteils (10A) die vorbestimmte Form enthält, die Anzeigesteuerung (68) eine Steuerung derart durchführt, dass das zweite Formbild, in dem der Abschnitt, der der vorbestimmten Form des Einsteckteils (10A) entspricht, gedreht ist, für jede gedrehte Drehposition angezeigt wird.

10. Vorrichtung zur Bildverarbeitung nach einem der Ansprüche 1 bis 9, wobei die erste Beobachtungsrichtung eine vorbestimmte Richtung als Beobachtungsrichtung zum Erfassen einer Gesamtform des Einsteckteils (10A) ist.

11. Vorrichtung zur Bildverarbeitung nach einem der Ansprüche 1 bis 10, wobei die Anzeigesteuerung (68) die Anzeigeeinheit (52) so steuert, dass sie ferner ein durch das Endoskop (10) erhaltenes endoskopisches Bild anzeigt.

12. Vorrichtung zur Bildverarbeitung nach einem der Ansprüche 1 bis 11, ferner umfassend:
eine Detektionseinheit (60), die die Form des in das Subjekt einzuführenden Einsteckteils (10A) in dem Endoskop (10) detektiert,
wobei die Erfassungseinheit (62) die Form des Einsteckteils (10A) von der Detektionseinheit (60) erfasst.

13. Ein Verfahren zur Bildverarbeitung, das von einem Computer ausgeführt wird, umfassend:
Erfassen einer Form eines Einsteckteils (10A), das in ein Subjekt in einem Endoskop (10) einzuführen ist, von einer Erfassungseinheit (62);
Erfassen einer vorbestimmten Form, die in einer Speichereinheit (76) gespeichert ist;
Bestimmen, ob die Form des Einsteckteils (10A) die vorbestimmte Form enthält oder nicht; und
Steuern einer Anzeigeeinheit (52), um ein erstes Formbild in einem Fall anzuzeigen, in dem bestimmt wird, dass die Form des Einsteckteils (10A) die vorbestimmte Form nicht enthält, und Steuern der Anzeigeeinheit (52), um das erste Formbild und ein zweites Formbild in einem Fall anzuzeigen, in dem bestimmt wird, dass die Form des Einsteckteils (10A) die vorbestimmte Form umfasst, wobei das erste Formbild die Form des Einsteckteils (10A) in einer ersten Beobachtungsrichtung repräsentiert, wobei das zweite Formbild einen Abschnitt umfasst, der der vorbestimmten Form des Einsteckteils (10A) in einer zweiten Beobachtungsrichtung entspricht, die sich von der ersten Beobachtungsrichtung unterscheidet.

14. Programm zur Bildverarbeitung, das einen Computer dazu veranlasst, einen Prozess auszuführen, der Folgendes umfasst:
Erfassen einer Form eines Einsteckteils (10A), das in ein Subjekt in einem Endoskop (10) einzuführen ist, von einer Erfassungseinheit (62);
Erfassen einer vorgegebenen Form, die in einer Speichereinheit (76) gespeichert ist;
Bestimmen, ob die Form des Einsteckteils (10A) die vorbestimmte Form enthält oder nicht; und
Steuern einer Anzeigeeinheit (52), um ein erstes Formbild in einem Fall anzuzeigen, in dem bestimmt wird, dass die Form des Einsteckteils (10A) die vorbestimmte Form nicht enthält, und Steuern der Anzeigeeinheit (52), um das erste Formbild und ein zweites Formbild in einem Fall anzuzeigen, in dem bestimmt wird, dass die Form des Einsteckteils (10A) die vorbestimmte Form umfasst, wobei das erste Formbild die Form des Einsteckteils (10A) in einer ersten Beobachtungsrichtung repräsentiert, wobei das zweite Formbild einen Abschnitt umfasst, der der vorbestimmten Form des Einsteckteils (10A) in einer zweiten Beobachtungsrichtung entspricht, die sich von der ersten Beobachtungsrichtung unterscheidet.

## Revendications

1. Dispositif de traitement d'images comprenant :
une unité d'acquisition (62) qui acquiert une forme d'une part d'insertion (10A) destinée à être insérée dans un sujet dans un endoscope (10) ;
une unité de stockage (76) dans laquelle est stockée une forme prédéterminée ;
une unité de détermination qui détermine si la forme de la pièce d'insertion (10A) inclut ou non la forme prédéterminée stockée dans l'unité de stockage (76) ; et **caractérisée par**
un contrôleur d'affichage (68) qui commande une unité d'affichage (52) pour afficher une première image de forme dans un cas où l'unité de détermination détermine que la forme de la part d'insertion (10A) n'inclut pas la forme prédéterminée, et commande l'unité d'affichage (52) pour afficher la première image de forme et une deuxième image de forme dans un cas où l'unité de détermination détermine que la forme de la part d'insertion (10A) inclut la forme prédéterminée, la première image de forme représentant la forme de la part d'insertion (10A) dans une première direction de point de vue, la deuxième image de forme incluant une partie correspondant à la forme prédéterminée de la part d'insertion (10A) dans une deuxième direction de point de vue différente de la première direction de point de vue.

2. Dispositif de traitement d'images selon la revendication 1, comprend en outre :
une unité de génération (64) qui génère la première image de forme dans un cas où l'unité de détermination détermine que la forme de la part d'insertion (10A) n'inclut pas la forme prédéterminée, et qui génère la première image de forme et la deuxième image de forme dans un cas où l'unité de détermination détermine que la forme de la part d'insertion (10A) inclut la forme prédéterminée, la première image de forme représentant la forme de la part d'insertion (10A) dans la première direction de point de vue, la deuxième image de forme incluant une portion correspondant à la forme prédéterminée de la part d'insertion (10A) dans la deuxième direction de point de vue différente de la première direction de point de vue.

3. Dispositif de traitement d'images selon la revendication 1 ou 2, dans lequel la forme prédéterminée est une forme de boucle.

4. Dispositif de traitement d'images selon la revendication 3, dans lequel la deuxième direction de point de vue est une direction de liaison d'un point d'intersection d'une portion en forme de boucle de la part d'insertion (10A) à un point central de la portion en forme de boucle.

5. Dispositif de traitement d'image selon la revendication 3, dans lequel la deuxième direction de point de vue est une direction prédéterminée comme direction de point de vue pour saisir un état d'intersection d'une portion en forme de boucle de la part d'insertion (10A).

6. Dispositif de traitement d'images selon l'une quelconque des revendications 1 à 5, dans lequel dans un cas où l'unité de détermination détermine que la forme de la part d'insertion (10A) inclut la forme prédéterminée, le contrôleur d'affichage (68) commande l'unité d'affichage (52) pour afficher la deuxième image de forme sur une région partielle dans la première image de forme.

7. Dispositif de traitement d'images selon l'une quelconque des revendications 1 à 6, dans lequel dans un cas où l'unité de détermination détermine que la forme de la part d'insertion (10A) inclut la forme prédéterminée, le contrôleur d'affichage (68) commande l'unité d'affichage (52) pour afficher en outre des informations qui sont visuellement reconnaissables dans la deuxième direction de point de vue.

8. Dispositif de traitement d'images selon l'une quelconque des revendications 1 à 7, dans lequel, dans un cas où l'unité de détermination détermine que la forme de la part d'insertion (10A) inclut la forme prédéterminée, le contrôleur d'affichage (68) effectue une commande telle que la deuxième image de forme dans laquelle la deuxième direction de point de vue est modifiée est affichée pour chaque deuxième direction de point de vue modifiée.

9. Le dispositif de traitement d'images selon l'une quelconque des revendications 1 à 7, dans lequel dans un cas où la forme de la part d'insertion (10A) inclut la forme prédéterminée, le contrôleur d'affichage (68) effectue une commande telle que la deuxième image de forme dans laquelle la portion correspondant à la forme prédéterminée de la part d'insertion (10A) est tournée est affichée pour chaque position de rotation tournée.

10. Dispositif de traitement d'images selon l'une quelconque des revendications 1 à 9, dans lequel la première direction de point de vue est une direction prédéterminée en tant que direction de point de vue pour saisir une forme globale de la part d'insertion (10A).

11. Dispositif de traitement d'images selon l'une quelconque des revendications 1 à 10, dans lequel le contrôleur d'affichage (68) contrôle l'unité d'affichage (52) pour afficher en outre une image endoscopique obtenue par l'endoscope (10).

12. Dispositif de traitement d'images selon l'une quelconque des revendications 1 à 11, comprend en outre :
une unité de détection (60) qui détecte la forme de la part d'insertion (10A) à insérer dans le sujet dans l'endoscope (10),
dans lequel l'unité d'acquisition (62) acquiert la forme de la part d'insertion (10A) à partir de l'unité de détection (60).Procédé de traitement d'images exécuté par un ordinateur, comprenant :
acquérir une forme d'une part d'insertion (10A) à insérer dans un sujet dans un endoscope (10) à partir d'une unité d'acquisition (62) ;
acquérir une forme prédéterminée stockée dans une unité de stockage (76) ;
déterminer si la forme de la part d'insertion (10A) inclut ou non la forme prédéterminée ; et
contrôler une unité d'affichage (52) pour afficher une première image de forme dans un cas où la détermination est faite que la forme de la part d'insertion (10A) n'inclut pas la forme prédéterminée, et contrôler l'unité d'affichage (52) pour afficher la première image de forme et une deuxième image de forme dans un cas où la détermination est faite que la forme de la part d'insertion (10A) inclut la forme prédéterminée, la première image de forme représentant la forme de la part d'insertion (10A) dans une première direction de point de vue, la deuxième image de forme incluant une portion correspondant à la forme prédéterminée de la part d'insertion (10A) dans une deuxième direction de point de vue différente de la première direction de point de vue.

13. Procédé de traitement d'images exécuté par un ordinateur, comprenant :
acquérir une forme d'une part d'insertion (10A) à insérer dans un sujet dans un endoscope (10) à partir d'une unité d'acquisition (62) ;
acquérir une forme prédéterminée stockée dans une unité de stockage (76) ;
déterminer si la forme de la part d'insertion (10A) inclut ou non la forme prédéterminée ; et
contrôler une unité d'affichage (52) pour afficher une première image de forme dans un cas où la détermination est faite que la forme de la part d'insertion (10A) n'inclut pas la forme prédéterminée, et contrôler l'unité d'affichage (52) pour afficher la première image de forme et une deuxième image de forme dans un cas où la détermination est faite que la forme de la part d'insertion (10A) inclut la forme prédéterminée, la première image de forme représentant la forme de la part d'insertion (10A) dans une première direction de point de vue, la deuxième image de forme incluant une portion correspondant à la forme prédéterminée de la part d'insertion (10A) dans une deuxième direction de point de vue différente de la première direction de point de vue.

14. Programme de traitement d'images permettant à un ordinateur d'exécuter un processus comprenant :
acquérir une forme d'une part d'insertion (10A) à insérer dans un sujet dans un endoscope (10) à partir d'une unité d'acquisition (62) ;
acquérir une forme prédéterminée stockée dans une unité de stockage (76) ;
déterminer si la forme de la part d'insertion (10A) inclut ou non la forme prédéterminée ; et
contrôler une unité d'affichage (52) pour afficher une première image de forme dans un cas où la détermination est faite que la forme de la part d'insertion (10A) n'inclut pas la forme prédéterminée, et contrôler l'unité d'affichage (52) pour afficher la première image de forme et une deuxième image de forme dans un cas où la détermination est faite que la forme de la part d'insertion (10A) inclut la forme prédéterminée, la première image de forme représentant la forme de la part d'insertion (10A) dans une première direction de point de vue, la deuxième image de forme incluant une portion correspondant à la forme prédéterminée de la part d'insertion (10A) dans une deuxième direction de point de vue différente de la première direction de point de vue.
